# FASCICULE DE BREVET EUROPEEN

(11) **EP 2 481 376 B1**
(45) Date de publication et mention de la délivrance du brevet: **23.04.2014**
(21) Numéro de dépôt: 12153346.7
(22) Date de dépôt: 31.01.2012
(51) Int. Cl.: A61F 2/40

(54) **Implant glénoïdien pour prothèse d'épaule et kit chirurgical**
Glenoidales Implantat für Schulterprothese, und entsprechender chirurgischer Kit
Glenoidal implant for shoulder prosthesis and surgical kit

(30) Priorité: 08.02.2011 FR 1150994; 01.02.2011 US 201161438570 P
(43) Date de publication de la demande: 01.08.2012
(73) Titulaire: Tornier, 38330 Montbonnot-Saint-Martin (FR)
(72) Inventeur: Burkhead JR., Wayne Z., Dallas, Texas 75231 (US); Boileau, Pascal, 06200 Nice (FR); Walch, Gilles, 69003 Lyon (FR); Deransart, Pierric, 38410 Saint Martin D'Uriage (FR)
(74) Mandataire: Grand, Guillaume

(56) Documents cités:
- EP-A1- 0 581 667
- FR-A1- 2 776 506
- US-A1- 2005 261 775
- US-A1- 2008 294 268

## Description

La présente invention concerne un implant glénoïdien pour prothèse d'épaule, ainsi qu'un kit chirurgical comprenant un tel implant glénoïdien.

De manière classique, une prothèse d'épaule comprend un implant glénoïdien, destiné à remplacer la cavité glénoïdale de l'omoplate, et/ou un implant huméral, destiné à remplacer la tête de l'humérus. L'implant glénoïdien est généralement composé d'un corps articulaire, destiné à s'articuler avec la tête de l'humérus, et de moyens de fixation pour solidariser le corps articulaire avec l'omoplate.

Le corps articulaire peut être métallique, par exemple en alliage de titane, en céramique, ou synthétique, par exemple en polyéthylène. Les matériaux synthétiques sont légèrement déformables, tandis que les alliages métalliques et les céramiques utilisés de manière classique sont relativement rigides. Par conséquent, les moyens de fixation doivent être adaptés au matériau du corps articulaire.

Un des buts de l'invention est de proposer des moyens de fixation adaptés à la fois aux corps articulaires métalliques et synthétiques, afin de minimiser le nombre de pièces que le chirurgien doit avoir en stock. De plus, des moyens de fixation mixtes permettent de réduire les coûts de conception et de fabrication des implants glénoïdiens, puisqu'un même moyen de fixation est adapté à plusieurs types de corps articulaires.

Un autre but de l'invention est de réduire la quantité de tissu osseux que le chirurgien doit enlever de la cavité glénoïdale pour fixer l'implant glénoïdien. De manière classique, les implants peu invasifs sont privilégiés car ils permettent d'augmenter la durée de vie de la prothèse d'épaule.

Dans ce contexte, US-A-2007/0219637 propose un implant glénoïdien dont la fixation à l'omoplate est réalisée au moyen d'un plot central qui est vissé, préalablement à la pose de l'implant, dans une douille surmoulée au niveau de la face arrière d'un corps articulaire de l'implant glénoïdien. Le blocage en rotation de l'implant par rapport à l'omoplate est assuré exclusivement par un plot latéral du corps de l'implant, ce qui n'est pas tout à fait satisfaisant puisque cela implique de creuser le tissu osseux pour loger le plot latéral, au détriment du respect de la constitution osseuse de la glène. De plus, cet implant est de conception relativement complexe puisqu'il nécessite de surmouler la douille.

US-A-2007/0055380 concerne un implant glénoïdien qui comprend un corps articulaire équipé de plusieurs pions de fixation latéraux, qui possèdent une certaine élasticité. Lors de l'opération, le chirurgien implante des éléments d'ancrage dans la glène. Les pions de fixation sont adaptés pour se clipper sur les éléments d'ancrage. Ainsi, ce mode de fixation n'est pas adapté aux corps articulaires métalliques, puisque les pions de fixation du corps articulaire doivent se déformer légèrement pour être fixés aux éléments d'ancrage. De plus, plusieurs pions de fixation sont nécessaires pour bloquer la rotation du corps articulaire par rapport à la glène, ce qui implique de creuser dans la glène plusieurs logements pour recevoir plusieurs éléments d'ancrage.

US-B-6406495 concerne un implant glénoïdien qui comprend un corps articulaire pourvu de plusieurs pions de fixation latéraux, destinés à être reçus dans des trous que le chirurgien perce préalablement dans la glène. Les pions de fixation sont filetés, et sont destinés à recevoir un manchon taraudé, pourvu extérieurement de nervures longitudinales. Lors de l'opération, le chirurgien visse préalablement des manchons sur les pions de fixation, puis il impacte l'ensemble dans la glène. Ici encore, plusieurs pions de fixation sont nécessaires pour bloquer la rotation du corps articulaire par rapport à la glène, ce qui détériore le tissu osseux de la glène et contribue à diminuer la durée de vie de l'implant.

Le document FR-A-2579454 propose un implant glénoïdien composé d'un corps articulaire fixé à la glène au moyen d'une douille métallique filetée, qui est vissée dans la glène et qui définit un logement tronconique. Le corps articulaire est pourvu d'un pion tronconique que le chirurgien emmanche dans le logement tronconique de la douille métallique. Cependant, la douille ne permet pas de bloquer la rotation du corps articulaire par rapport à l'omoplate car elle a tendance à se dévisser de la glène. Pour bloquer la rotation de l'implant glénoïdien, le corps articulaire est assemblé à l'omoplate au moyen de deux pattes de fixation latérales, vissées de part et d'autre de l'omoplate, ce rend cet implant relativement invasif, et complique la pose de l'implant.

US-A-2007/0260321 divulgue un élément de fixation destiné à fixer un corps articulaire dans la glène. L'élément de fixation est en forme de manchon tronconique creux, pourvu extérieurement de nervures longitudinales ou d'ardillons (désignés par le terme anglais « barbs » dans le texte). Les nervures ou les ardillons permettent de bloquer l'élément de fixation dans la glène. Une vis de fixation est passée à travers l'élément de fixation et permet de l'immobiliser de manière plus efficace. L'élément de fixation est traversé par une ouverture longitudinale tronconique, dans laquelle sont ménagées deux rainures longitudinales. Le corps articulaire est pourvu d'un pion tronconique équipé de nervures longitudinales et est emmanché dans l'ouverture tronconique de l'élément de fixation, en positionnant les nervures dans les rainures pour bloquer la rotation du corps articulaire par rapport à l'élément de fixation. Cependant, cet élément de fixation n'est pas adapté pour recevoir un corps articulaire synthétique, car une fixation par cône d'emmanchement n'est pas efficace pour retenir un corps articulaire synthétique dans l'élément de fixation.

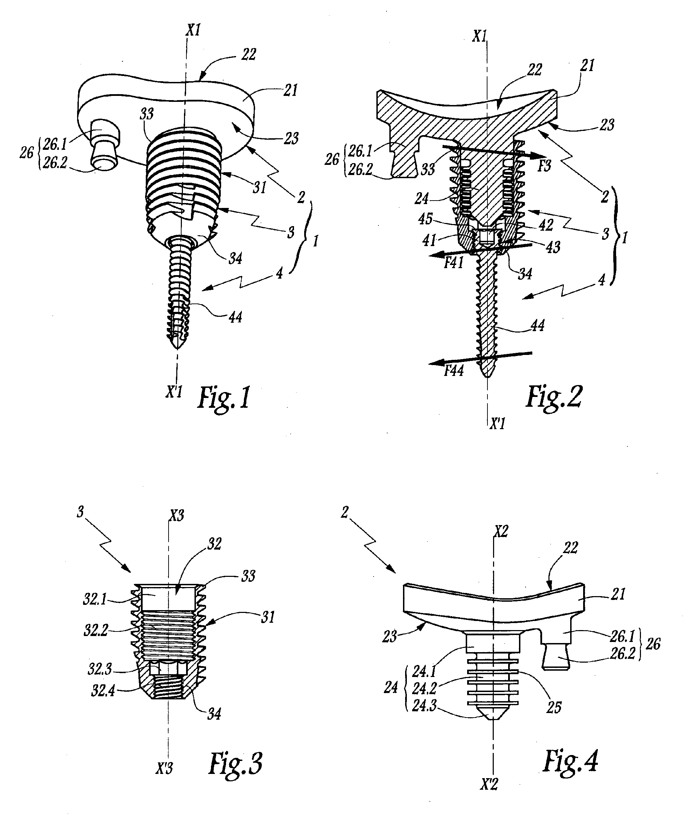

Le document US-A1-2008/0294268, sur lequel est basé le préambule de la revendication 1, divulgue un implant glénoïdien qui comprend un corps articulaire anatomique ou inversé, monté sur un élément central équipé de moyens d'ancrage dans la glène. Le corps articulaire anatomique ou inversé comprend un plateau dont une face, destinée à être tournée vers la glène, est pourvue d'un pion pour l'assemblage du corps articulaire avec un pion de l'élément central. Dans le mode de réalisation de la figure 3, l'assemblage entre le pion du corps articulaire et le pion de l'élément central est réalisé par emmanchement conique. L'implant glénoïdien comprend en outre des moyens de blocage de la rotation de l'élément central par rapport à la glène. Dans le mode de réalisation de la figure 3, ces moyens de blocage sont constitués par des vis de fixation de l'élément central dans la glène ainsi que par des ailettes longitudinales du pion central prévues pour s'ancrer dans la glène. Ainsi, le corps articulaire n'est pas bloqué en rotation par rapport à la glène, ce qui compromet la stabilité de l'implant et peut conduire à son descellement.

Les documents US-A1-2005/0261775, EP-A1-0 581 667 et FR-A1-2 776 506 divulguent d'autres réalisations d'implants glénoïdiens pour prothèse d'épaule.

Dans le but de remédier aux inconvénients cités ci-dessus, l'invention a pour objet un implant glénoïdien pour prothèse d'épaule tel que défini à la revendication 1.

Grâce à l'invention, le blocage en rotation de l'implant glénoïdien par rapport à l'omoplate est assuré sans que le chirurgien ne doive creuser un trou latéral supplémentaire dans la glène, destiné notamment à recevoir un plot latéral du corps articulaire. De plus, l'élément central est adapté pour recevoir indifféremment un corps articulaire synthétique ou un corps articulaire métallique ou céramique.

Des aspects avantageux mais non obligatoires de l'invention sont spécifiés aux revendications dépendantes 2 à 11.

L'invention a également pour objet un kit chirurgical tel que défini à la revendication 12.

L'invention sera mieux comprise à la lecture de la description qui va suivre, donnée uniquement à titre d'exemple et faite en référence aux dessins sur lesquels :
- la figure 1 est une vue en perspective d'un implant glénoïdien conforme à l'invention ;
- la figure 2 est une coupe axiale de l'implant glénoïdien de la figure 1 ;
- la figure 3 est une coupe axiale d'un élément central de l'implant glénoïdien de la figure 1 ;
- la figure 4 est une vue latérale d'un corps articulaire de l'implant glénoïdien de la figure 1 ;
- la figure 5 est une vue en perspective d'un implant glénoïdien conforme à un deuxième mode de réalisation de l'invention ;
- la figure 6 est une coupe axiale de l'implant glénoïdien de la figure 5 ;
- la figure 7 est une coupe axiale d'un élément central de l'implant glénoïdien de la figure 5 ;
- la figure 8 est une vue en perspective d'un corps articulaire de l'implant glénoïdien de la figure 5 ;
- la figure 9 est une vue éclatée en perspective d'un implant glénoïdien ;
- la figure 10 est une coupe axiale en perspective de l'implant glénoïdien de la figure 9 ;
- la figure 11 est une vue éclatée en perspective d'un implant glénoïdien ;
- la figure 12 est une coupe axiale en perspective de l'implant glénoïdien de la figure 11 ;
- la figure 13 est une vue semblable à la figure 10 qui montre l'implant glénoïdien de la figure 9, superposé avec un corps articulaire de l'implant glénoïdien de la figure 11 ;
- la figure 14 est une coupe axiale de l'implant glénoïdien de la figure 9, superposé avec le corps articulaire de l'implant glénoïdien de la figure 11 ;
- la figure 15 est une vue éclatée en perspective d'un implant glénoïdien ;
- la figure 16 est une coupe axiale, en perspective, de l'implant glénoïdien de la figure 15;
- la figure 17 est une vue en perspective d'un implant glénoïdien ; et
- la figure 18 est une coupe axiale de l'implant glénoïdien de la figure 17.

Les figures 1 à 4 montrent un implant glénoïdien 1 dit anatomique ou non inversé, destiné à être implanté dans la glène de l'omoplate, non représentée, d'un être humain. L'implant glénoïdien 1 est composé d'un corps articulaire 2, d'un élément central 3 pour l'ancrage du corps articulaire 2 dans la glène de l'omoplate, et d'une vis 4 de blocage en rotation de l'élément central 3 par rapport à l'omoplate. Le corps articulaire 2, après avoir été implanté dans la glène, est adapté pour s'articuler avec une tête, éventuellement prothésée, de l'humérus, non représenté, du patient opéré, en vue de reproduire un comportement articulaire aussi proche que possible du comportement d'origine de l'épaule du patient.

Dans la suite, les qualificatifs « supérieur » et « inférieur » font référence à l'orientation de l'implant glénoïdien 1 à la figure 1, où le corps articulaire 2 est en haut d'un axe géométrique longitudinal X1-X'1 de l'implant 1, tandis que la vis 4 est en bas de l'axe X1-X'1.

Le corps articulaire 2, visible plus particulièrement à la figure 4, comprend un plateau 21 globalement perpendiculaire à un axe géométrique longitudinal X2-X'2 du corps articulaire 2. Le plateau 21 délimite une face supérieure 22 concave, qui constitue une surface articulaire pour l'articulation de l'omoplate avec la tête de l'humérus, et par une face inférieure 23, équipée d'un pion central 24, qui s'étend le long de l'axe X2-X'2. Le pion central 24 comprend une partie supérieure 24.1 cylindrique à section circulaire, une partie intermédiaire 24.2 cylindrique à section circulaire, de diamètre inférieur à la partie supérieure, et une partie inférieure 24.3 tronconique, dont le diamètre diminue en s'éloignant du plateau 21. La partie intermédiaire 24.2 est pourvue de cinq ailettes 25 perpendiculaires à l'axe X2-X'2 et parallèles entre elles, qui sont réparties le long de la partie centrale 24.2 et qui s'étendent chacune sur la périphérie de la partie intermédiaire 24.2.

Le corps articulaire 2 est constitué d'un ou deux matériaux synthétiques, comme par exemple le polyéthylène, le PEEK ou le polyuréthane, ce qui confère aux ailettes 25 une élasticité leur permettant de se déformer légèrement.

La face inférieure 23 est également équipée d'un plot latéral 26 parallèle à l'axe X2-X'2. Le plot latéral 26 comprend une partie supérieure 26.1 qui est reliée à la face inférieure 23 du plateau 21 et une partie inférieure 26.2 reliée à la partie supérieure 26.1. La partie supérieure 26.1 est cylindrique à section circulaire, et son diamètre est inférieur au diamètre du pion central 24. La partie inférieure 26.2 est tronconique, et son diamètre augmente en s'éloignant du plateau 21.

L'élément central 3, visible plus particulièrement à la figure 3, est globalement en forme de cylindre creux à section circulaire, d'axe géométrique longitudinal X3-X'3. L'élément central 3 est délimité par une surface latérale externe 31 qui porte un filetage auto-taraudeur, et est traversé longitudinalement par une ouverture 32, centrée sur l'axe longitudinal X3-X'3, qui s'étend entre une extrémité supérieure 33 et une extrémité inférieure 34 de l'élément central 3. L'extrémité inférieure 34 de l'élément central 3 est tronconique et son diamètre diminue en se rapprochant de l'extrémité inférieure 34.

L'ouverture 32 comprend une partie supérieure 32.1 cylindrique à section circulaire, une partie taraudée 32.2, de diamètre similaire à la partie supérieure, une partie de prise d'outil 32.3, de section transversale hexagonale, et une partie inférieure 32.4 cylindrique à section circulaire, taraudée et de plus petit diamètre que la partie supérieure 32.1.

L'élément central 3 est fabriqué à partir d'un alliage métallique ou de céramique biocompatible et sa rigidité est plus importante que celle du corps articulaire 2. Par exemple, l'élément central 3 peut être fabriqué à partir d'acier inoxydable, de titane ou alliage de titane, d'un alliage cobalt-chrome ou de PEEK. L'élément central 3 n'est pas prévu pour, en service, se déformer.

La section transversale maximale de l'élément central 3, prise perpendiculairement à l'axe longitudinal X3-X'3, est inférieure à la section transversale minimale du plateau 21 du corps articulaire 2, prise perpendiculairement à l'axe longitudinal X2-X'2. La partie de plus grande section de l'élément central 3 est la partie supérieure filetée.

Le contact entre l'élément central 3 et le corps articulaire 2 est réalisé uniquement sur une partie du corps articulaire 2, en l'occurrence sur le pion central 24. L'élément central 3 n'est pas en contact avec la totalité de la surface inférieure 23 du plateau 21.

De préférence, la section transversale du plateau 21 est entre 1,8 à 5 fois supérieure à la section transversale maximale de l'élément central 3, de préférence encore 2,2 fois supérieure à la section transversale maximale de l'élément central 3.

L'élément central 3 assure l'ancrage du corps articulaire 2 dans la glène via le pion central 24, mais l'élément central 3 ne soutient pas le plateau 21.

La vis 4, d'axe géométrique longitudinal X4-X'4, comprend une tête 41 et une tige 44 filetée auto-taraudante. Un trou borgne 42 à section hexagonale, centré sur l'axe X4-X'4, est réalisé dans la tête 41 de la vis 4. Une surface latérale externe 43 de la tête 41 est filetée, et est prolongée vers le haut par une collerette 45 de plus grand diamètre que la partie filetée. La vis 4 est fabriquée à partir d'un matériau métallique ou de céramique biocompatible, tel qu'un acier inoxydable, du titane, un alliage de titane ou un alliage de cobalt-chrome.

On dit qu'un filetage ou un taraudage est « à droite » lorsque le vissage s'effectue dans le sens antihoraire, et qu'un filetage ou un taraudage est « à gauche » lorsque le vissage s'effectue dans le sens horaire.

Le filetage de la surface latérale externe 31 de l'élément central 3 est à droite, comme représenté par la flèche F3. Le filetage de la tige 44 de la vis 4 est à gauche, comme représenté par la flèche F44, et le filetage de la tête 41 de la vis 4 et le taraudage de la partie inférieure 32.4 de l'ouverture 32 sont à gauche, comme représenté par la flèche F41.

Lorsqu'il convient de réaliser la pose de l'implant glénoïdien 1 dans la glène de l'omoplate, le chirurgien perce préalablement un trou dans la glène, de diamètre légèrement inférieur au diamètre externe de l'élément central 3. Puis, le chirurgien visse l'élément central 3 dans la glène, au moyen d'un outil, non représenté, équipé d'un embout à section hexagonale qu'il insère dans la partie de prise d'outil 32.3 de l'ouverture 32. Le filetage auto-taraudant de l'élément central 3 permet à celui-ci de pénétrer dans la glène, plus précisément dans le trou préalablement réalisé par le chirurgien, en le taraudant. L'élément central 3 permet à lui seul de fixer le corps articulaire 2 dans la glène.

Puis, le chirurgien fait un trou dans la glène, de diamètre légèrement inférieur au diamètre de la tige 44 de la vis 4, en passant la mèche d'un moteur chirurgical à travers l'ouverture 32 de l'élément central 3. La mèche est guidée par un outil, non représenté, en prise dans une des parties de l'ouverture 32 et protégeant le taraudage 32.4. Le chirurgien visse ensuite la tête 41 de la vis 4 dans ce trou, à l'aide d'un outil, non représenté, équipé d'un embout à section hexagonale qu'il insère dans le trou borgne 42 de la vis 4. Le filetage de la tête 41 de la vis 4 coopère avec le taraudage de la partie inférieure 32.4 de l'ouverture 32. L'axe X3-X'3 de l'élément central 3 est alors confondu avec l'axe X4-X'4 de la vis 4. Le filetage de la tête 41 de la vis 4 est dans le même sens que le filetage de la tige 44 de la vis 4, ce qui permet au chirurgien, dans un même mouvement, de visser tête 41 dans la partie inférieure 32.4 de l'ouverture 32 et de visser la tige 44 dans le trou qu'il vient de percer dans la glène. Le filetage auto-taraudant de la tige 44 de la vis 4 améliore l'ancrage de la vis 4 dans la glène. En fin de course, la collerette 45 de la tête 41 de la vis 4, qui est de plus grand diamètre que la partie inférieure 32.4 de l'ouverture 32, permet de retenir la tête 41 de la vis 4 dans l'ouverture 32 en l'empêchant de passer à travers l'ouverture 32.

De manière avantageuse mais non obligatoire, le filetage de la tête 41 de la vis 4 comporte deux filets, pour faciliter la prise de ce filetage dans le taraudage de la partie inférieure 32.4 de l'ouverture 32. De plus, de manière avantageuse mais non obligatoire, le pas du filetage de la tête 41 de la vis 4 est égal au pas du filetage de la tige 44 de la vis 4, pour optimiser la pénétration de la vis 4 dans la glène lorsque le filetage de la tête 41 de la vis 4 coopère avec le taraudage de la partie inférieure 32.4 de l'ouverture 32.

De manière avantageuse mais non obligatoire, le filetage de la tête 41 de la vis 4 comporte un frein, tel que par exemple un pion transverse en matière synthétique, pour éviter un dévissage ultérieur de la vis 4.

Une fois l'élément central 3 et la vis 4 en place dans la glène, le sous-ensemble formé par ces deux éléments est bloqué en rotation, par rapport à la glène, autour des axes X3-X'3 et X4-X'4, grâce au sens opposé du filetage de la surface latérale externe 31 de l'élément central 3, par rapport au sens du filetage de la tige 44 de la vis 4. En effet, lorsque ce sous-ensemble tend à se dévisser dans le sens du filetage de la surface latérale externe 31 de l'élément central 3, la rotation est bloquée par le filetage de la tige de la vis 44. Au contraire, lorsque ce sous-ensemble tend à se dévisser dans le sens du filetage de la tige 44 de la vis 4, la rotation est bloquée par le filetage de la surface latérale externe 31 de l'élément central 3.

Avant ou après avoir mis en place l'élément central 3 et la vis 4, le chirurgien perce un trou latéral dans la glène, destiné à recevoir le plot latéral 26 du corps articulaire 2.

Une fois l'élément central 3 et la vis 4 en place dans la glène, le chirurgien enfonce le pion central 24 du corps articulaire 2 dans l'ouverture 32 de l'élément central 3, jusqu'à ce que la face inférieure 23 du plateau 21 vienne en appui contre l'extrémité supérieure 33 de l'élément central 3. Les axes X2-X'2, X3-X'3 et X4-X'4 sont alors confondus avec l'axe X1-X'1 de l'implant glénoïdien 1.

Dans une alternative avantageuse, le chirurgien enfonce le pion central 24 dans l'ouverture 32, jusqu'à ce que la face inférieure 23 du plateau 1 vienne en appui contre la glène, sans que la face inférieure 23 soit en contact avec l'extrémité supérieure 33 de l'élément central 3. Ceci limite l'usure du corps articulaire 2 contre l'élément central 3.

La partie supérieure 24.1 du pion central est alors logée dans la partie supérieure 32.1 de l'ouverture 32, et la partie intermédiaire 24.2 du pion central 24 est alors logée dans la partie taraudée 32.2 de l'ouverture 32. Lorsque le chirurgien enfonce le pion central 24 dans l'ouverture 32, les ailettes 25 du pion central 24 se déforment au contact des filets du taraudage de la partie taraudée 32.2 de l'ouverture 32, et les ailettes 25 sont alors retenues entre ces filets, par coopération de formes et déformation souple. Le plot latéral 26 se trouve emprisonné dans le trou correspondant percé dans la glène et empêche la rotation, autour de l'axe X1-X'1, du corps articulaire 2 par rapport à la glène.

Les ailettes 25 empêchent la translation du corps articulaire 2, le long de l'axe X1-X'1, par rapport à l'élément central 3, lorsque l'intensité des efforts qui sont appliqués à l'implant glénoïdien 1 ne dépassent pas de manière trop importante l'intensité des efforts que subit naturellement l'articulation de l'épaule. Toutefois, si le chirurgien souhaite séparer le corps articulaire 2 et l'élément central 3, il est possible de retirer le corps articulaire 2 de l'élément central 3 à l'aide d'un outil approprié, tel qu'une pince.

Une fois que l'implant glénoïdien 1 est implanté dans la glène, sa rotation, autour de l'axe X1-X'1, par rapport à la glène, est bloquée. En effet, le plot latéral 26 bloque la rotation du corps articulaire 2 par rapport à la glène, et la rotation, par rapport à la glène, du sous-ensemble formé par l'élément central 3 et la vis 4, est bloquée grâce aux filetages en sens contraires de l'élément central 3 et de la tige 44 de la vis 4.

Le blocage de la rotation de l'implant glénoïdien 1 par rapport à la glène est primordial, car il empêche l'implant glénoïdien 1 de se desceller sous l'action des contraintes mécaniques résultant de l'appui et des mouvements de la tête de l'humérus contre la face supérieure 22 du corps articulaire 2.

Le plot latéral 26, qui permet le blocage en rotation, autour de l'axe X1-X'1, du corps articulaire 2 par rapport à la glène, est optionnel et peut être substitué par d'autres moyens de blocage en rotation. Par exemple, l'extrémité supérieure de la surface latérale externe du plot central 24 pourra être prévue pour dépasser axialement de l'élément central 3, et sa section transversale pourra être prévue, par exemple, carrée ou hexagonale, de manière à bloquer la rotation du corps articulaire 2 par rapport à la glène, une fois le pion central implanté dans la glène. Dans ce cas, le chirurgien creuse une seule cavité dans la glène, composée d'une partie de réception de l'élément central, alignée avec une partie de réception de la vis. Ceci est particulièrement avantageux car le tissu osseux est alors en grande partie préservé.

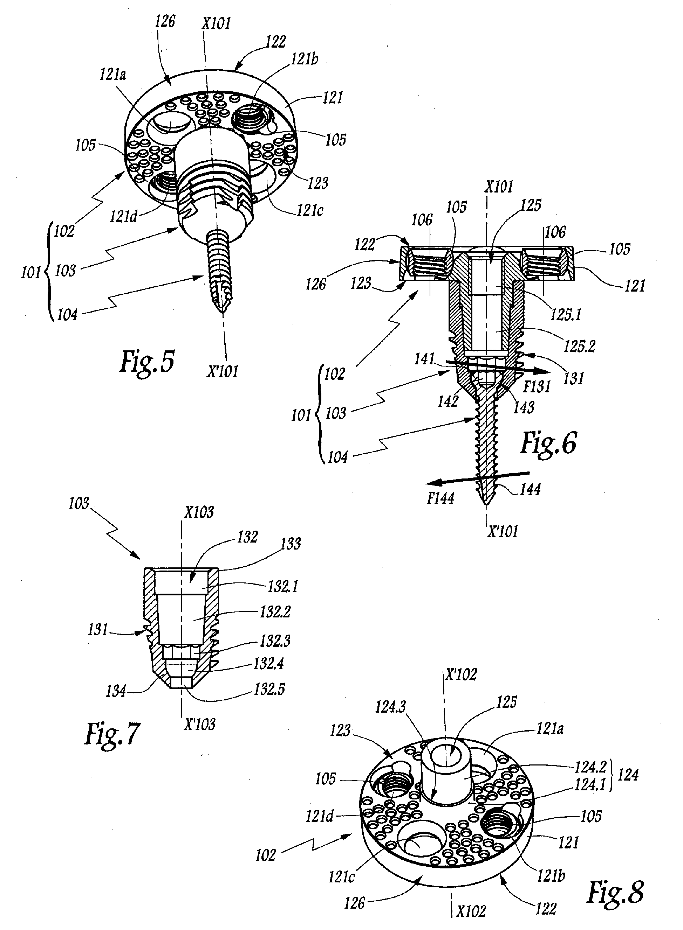

Les figures 5 à 8 représentent un implant glénoïdien 101, conforme à un deuxième mode de réalisation de l'invention, dont les éléments semblables à ceux du premier mode de réalisation portent les mêmes références, augmentées de 100.

Ainsi, l'implant glénoïdien 101, d'axe longitudinal X101-X'101, est composé d'un corps articulaire 102 de type « metal back », d'un élément central 103 pour l'ancrage du corps articulaire 102 dans la glène de l'omoplate, et d'une vis 104 de blocage en rotation de l'élément central 103 par rapport à l'omoplate.

Le corps articulaire 102, représenté en vue de dessous à la figure 8, comprend un plateau 121 à section circulaire, perpendiculaire à un axe longitudinal X102-X'102 du corps articulaire 102. Le plateau 121 est délimité par une face supérieure 122 et par une face inférieure 123. On note 126, une surface latérale tronconique du plateau 121.

La face supérieure destinée à recevoir une pièce supplémentaire, non représentée, adaptée pour s'articuler avec l'humérus. Dans le cas d'une prothèse inversée, cette pièce supplémentaire est hémisphérique, et un implant comprenant une surface articulaire concave pourra être implanté dans l'humérus.

Quatre logements 121 a, 121 b, 121 c et 121 d en forme de tronçon de sphère sont creusés dans le plateau 121 et débouchent à la fois sur la face supérieure 122 et sur la face inférieure 123.

Deux douilles 105 présentent une surface externe sphérique et sont reçues dans les logements 121b et 121d. Les douilles 105 sont en liaison rotule avec le corps articulaire 102. Les douilles 105 sont traversées chacune par un trou taraudé destiné à recevoir une vis 106. Les vis 106 sont représentées uniquement par leur trait d'axe à la figure 6.

La face inférieure 123 du plateau 121 est équipée d'un pion central 124, d'axe longitudinal X102-X'102, qui comprend une partie supérieure 124.1 cylindrique à section circulaire, reliée à la face inférieure 123, et une partie inférieure 124.2 tronconique, dont le diamètre diminue en s'éloignant du plateau 121.

Le corps articulaire 102 est traversé longitudinalement par une ouverture débouchante 125, centrée sur l'axe X102-X'102, qui comprend une partie supérieure 125.1 taraudée, et une partie inférieure 125.2 cylindrique à section circulaire. Le taraudage de la partie supérieure 125.1 est prévu pour la fixation au corps articulaire 102 de la pièce supplémentaire destinée à s'articuler avec la tête de l'humérus.

Le corps articulaire 102 est métallique ou en céramique. Par exemple, le corps articulaire 102 peut être fabriqué à partir d'acier inoxydable, de titane, d'alliage de titane ou de cobalt-chrome.

L'élément central 103, visible plus particulièrement à la figure 7, est globalement en forme de cylindre creux à section circulaire, d'axe longitudinal X103-X'103. L'élément central 103 est délimité par une surface latérale externe 131 qui porte un filetage auto-taraudant, et est traversé longitudinalement par une ouverture 132 qui s'étend entre une extrémité supérieure 133 et une extrémité inférieure 134 de l'élément central 103. L'extrémité inférieure 134 de l'élément central 3 est tronconique et son diamètre diminue en se rapprochant de l'extrémité inférieure 134.

L'ouverture 132 comprend une partie supérieure 132.1 cylindrique, de section circulaire, une partie tronconique 132.2, dont le diamètre diminue en se rapprochant de l'extrémité inférieure 134, une partie hexagonale 132.3, dont la section transversale est en forme d'hexagone, une partie arrondie 132.4 en forme de dôme, dont le sommet est tourné vers le bas, et une partie inférieure 132.5 cylindrique, de section circulaire.

L'élément central 103 est métallique ou en céramique. Par exemple, l'élément central 103 peut être fabriqué à partir d'acier inoxydable, de titane, d'alliage de titane ou de cobalt-chrome.

La section transversale maximale de l'élément central 103, prise perpendiculairement à l'axe longitudinal X103-X'103, est inférieure à la section transversale du plateau 121 du corps articulaire 102, prise perpendiculairement à l'axe longitudinal X102-X'102. La section transversale maximale de l'élément central 103 se situe au niveau de son extrémité supérieure 133. Le contact entre l'élément central 103 et le corps articulaire 102 est réalisé uniquement entre une partie du corps articulaire 102, en l'occurrence sur le pion central 124, et une zone annulaire de la surface inférieure 123 entourant le pion central 124. L'élément central 103 n'est pas en contact avec la totalité de la surface inférieure 123 du plateau 121. La zone annulaire d'appui du plateau 121 sur l'élément central 3 est de dimensions limitées vis-à-vis des dimensions globales de la surface inférieure 123.

L'élément central 103 assure l'ancrage du corps articulaire 102 dans la glène, et l'élément central 103 ne soutient pas le plateau 121.

La vis 104, d'axe longitudinal X104-X'104, comprend une tête 141 et une tige filetée 144 auto-taraudante. Un trou borgne 142 à section carrée, centré sur l'axe longitudinal X104-X'104, est réalisé dans la tête 141 de la vis 104. Une surface latérale externe 143 de la tête 141 est en forme d'un dôme dont le sommet est tourné vers le bas et dont la géométrie est complémentaire de celle de la partie arrondie 132.4 de l'ouverture 132 de l'élément central 103.

La vis 104 est métallique ou en céramique. Par exemple, la vis 104 peut être fabriquée à partir d'acier inoxydable, de titane, d'alliage de titane ou de cobalt-chrome.

Le filetage de la surface latérale externe 131 de l'élément central 103 est à droite, comme représenté par la flèche F131, tandis que le filetage de la tige 144 de la vis 104 est à gauche, comme représenté par la flèche F144.

Lorsqu'il convient de réaliser la pose de l'implant glénoïdien 101 dans la glène de l'omoplate, le chirurgien perce préalablement un trou dans la glène, de diamètre légèrement inférieur au diamètre externe de l'élément central 103. Puis, le chirurgien visse l'élément central 103 dans la glène, au moyen d'un outil, non représenté, équipé d'un embout à section hexagonale qu'il insère dans la partie hexagonale 132.3. Le filetage auto-taraudant de l'élément central 103 permet à celui-ci de pénétrer dans la glène en la taraudant. Le chirurgien fait un trou dans la glène, de diamètre légèrement inférieur au diamètre de la tige 144 de la vis 104, en passant une mèche, non représentée, à travers l'ouverture 132 de l'élément central 103. La mèche est guidée par un outil, non représenté, en prise dans une des parties de l'ouverture 132. Le chirurgien visse ensuite la vis 104 dans le trou qu'il vient de réaliser, à l'aide d'un outil, non représenté, équipé d'un embout à section carrée qu'il insère dans le trou borgne 142 de la vis 104. En fin de course, la surface latérale externe de la tête 141 de la vis 104 est en appui contre la surface latérale de la partie 132.4 de l'ouverture 132 de l'élément central 103.

Une fois l'élément central 103 et la vis 104 implantés dans la glène, le sous-ensemble formé par ces deux éléments est bloqué en rotation, par rapport à l'omoplate, grâce au sens opposé du filetage de la surface externe 131 de l'élément central 103, par rapport au sens du filetage de la tige 144 de la vis 104.

Une fois l'élément central 103 et la vis 104 en place dans la glène, le chirurgien enfonce le pion central 124 du corps articulaire 102 dans l'ouverture 132 de l'élément central 103, de sorte que la partie inférieure 124.2 tronconique du pion central 124 est coincée dans la partie tronconique 132.2 de l'ouverture 132. Cet assemblage de type « cône morse » permet de bloquer la translation et la rotation du corps articulaire 102 par rapport à l'élément central 103, lorsque l'intensité des efforts qui leur sont appliqués ne dépasse pas outre mesure celle des efforts que subit naturellement une articulation.

Toutefois, s'il convient de retirer l'implant glénoïdien 101 de l'omoplate, le chirurgien, à l'aide d'outils appropriés, peut désemmancher le corps articulaire 102 de l'élément central 103.

Les vis 106 sont prévues pour fixer de manière plus solide le corps articulaire 102 à la glène et bloquer ainsi la rotation du corps articulaire 102 par rapport à la glène. La rotation des douilles 105 dans les logements 121 b et 121 d permet d'orienter les vis 106 de manière appropriée.

Le corps articulaire 102 peut, en variante, être fixé à l'élément central 103 avant l'insertion de la vis 104 ou avant le vissage de l'élément central 103 dans la glène.

Enfin, le chirurgien fixe au corps articulaire 102 la pièce supplémentaire, destinée à s'articuler avec la tête de l'humérus. Cette pièce supplémentaire est vissée dans le taraudage de la partie supérieure 125.1 du logement 125. La pièce supplémentaire comprend un évidement tronconique prévu pour s'emmancher sur la surface latérale 126 du plateau 121.

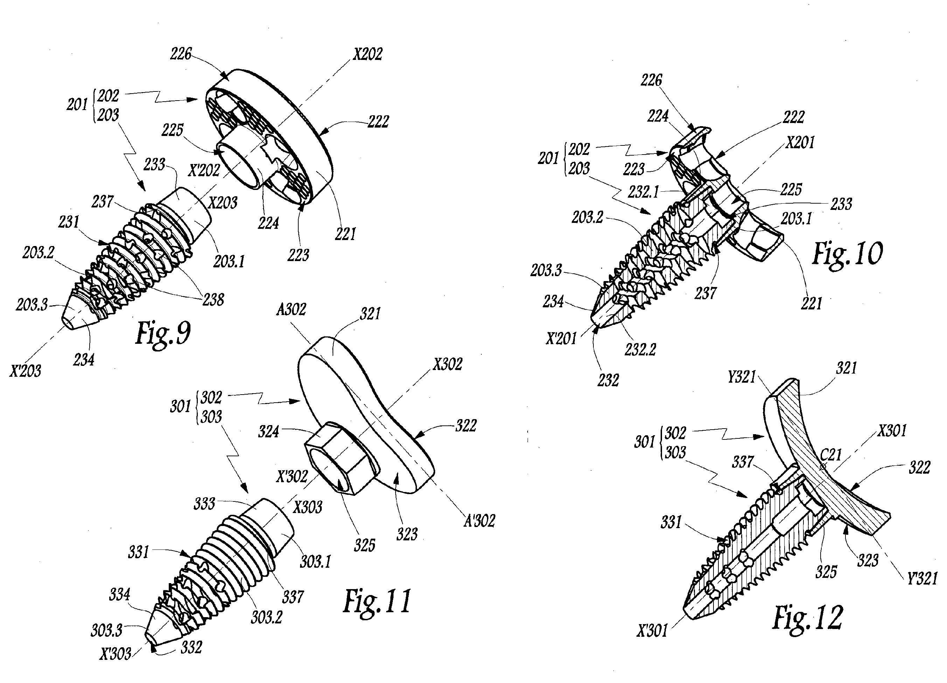

Les figures 9 et 10 représentent un implant glénoïdien 201, dont les éléments analogues à ceux du premier mode de réalisation portent les mêmes références, augmentées de 200.

Ainsi, l'implant glénoïdien 201, d'axe longitudinal X201-X'201, est composé d'un corps articulaire 202, de type « metal back », et d'un élément central 203 pour l'ancrage du corps articulaire 202 dans la glène de l'omoplate.

Le corps articulaire 202, d'axe longitudinal X202-X'202, comprend un plateau 221 circulaire, perpendiculaire à l'axe X202-X'202, qui est délimité par une face supérieure 222 et par une face inférieure 223. On note 226, une surface latérale du plateau 221. La face inférieure 223 est équipée d'un pion central 224 cylindrique à section circulaire, centré sur l'axe X202-X'202. Le pion central 224 est creux et définit un logement 225 tronconique, dont le diamètre diminue en se rapprochant du plateau 221.

La face supérieure 222 du plateau 221 est destinée à recevoir une pièce supplémentaire, non représentée, apte à s'articuler avec la tête de l'humérus.

L'élément central 203, d'axe longitudinal X203-X'203, comprend une partie intermédiaire 203.2 tronconique dont le diamètre diminue entre une extrémité supérieure 233 et une extrémité inférieure 234 de l'élément central 203. Une surface latérale externe 231 de la partie intermédiaire 203.2 porte un filetage auto-taraudant et est traversée transversalement par des trous 238. La partie intermédiaire 203.2 est prolongée vers le bas par une portion inférieure 203.3 tronconique, dont la conicité est plus importante que celle de la partie intermédiaire 203.2. La partie intermédiaire 203.2 est prolongée vers le haut par une partie supérieure 203.1 tronconique, dont le diamètre diminue en se rapprochant de l'extrémité supérieure 233. Une rainure périphérique 237 est définie entre la partie intermédiaire 203.2 et la partie supérieure 203.1

L'élément central 203 est traversé par une ouverture longitudinale 232, centrée sur l'axe X203-X'203, qui débouche de part et d'autre de l'élément central 203. L'ouverture 232 est composée d'une partie supérieure 232.1, de section transversale hexagonale, et d'une partie inférieure 232.2 cylindrique à section circulaire qui relie la partie supérieure 232.1 à l'extrémité inférieure 234 de l'élément central 203.

Les trous 238 sont répartis sur toute la portion intermédiaire 203.2 et débouchent dans la partie inférieure 232.2 de l'ouverture 232.

La section transversale maximale de l'élément central 203, prise perpendiculairement à l'axe longitudinal X203-X'203, est inférieure à la section transversale du plateau du corps articulaire 202, prise perpendiculairement à l'axe longitudinal X202-X'202. Le contact entre l'élément central 203 et le corps articulaire 202 est réalisé uniquement sur une partie du corps articulaire 202, en l'occurrence sur le pion central 224. L'élément central 203 n'est pas en contact avec la totalité de la surface inférieure 223 du plateau 221. L'élément central 203 assure l'ancrage du corps articulaire 202 dans la glène, et l'élément central 203 ne soutient pas la surface inférieure 223 du plateau 221.

Lorsqu'il convient de réaliser la pose de l'implant glénoïdien 201 dans l'omoplate, le chirurgien perce préalablement un trou dans la glène, de diamètre légèrement inférieur au diamètre de l'élément central. Puis, le chirurgien visse l'élément central 203 dans la glène, au moyen d'un outil, non représenté, équipé d'un embout à section hexagonale qu'il insère dans la partie supérieure 232.1 de l'ouverture 232.

Le corps articulaire 202 est assemblé à l'élément central 203, en emmanchant le pion central 224 sur la partie supérieure 203.1 de l'élément central 203. Lorsque le corps articulaire 202 est assemblé à l'élément central 203, le corps articulaire 202 est bloqué en translation et en rotation par rapport à l'élément central 203 grâce à la fixation de type « cône-morse » entre ces deux éléments.

Une fois l'implant glénoïdien 201 posé, le tissu osseux de la glène colonise les trous 238 de l'élément central 203 et l'ouverture 232, ce qui contribue à bloquer la rotation de l'élément central 203 par rapport à la glène, et améliore la tenue de l'implant glénoïdien 201. La rotation du corps articulaire 202 par rapport à la glène est bloquée au moyen de vis et de douilles non représentées, semblables aux vis 106 et aux douilles 105 du deuxième mode de réalisation.

Les figures 11 et 12 représentent un implant glénoïdien 301, dont les éléments analogues à ceux du troisième mode portent les mêmes références, augmentées de 100.

L'implant glénoïdien 301, d'axe longitudinal X301-X'301, est composé d'un corps articulaire 302 et d'un élément central 303 pour l'ancrage du corps articulaire 302 dans la glène de l'omoplate.

Le corps articulaire 302, après sa fixation à la glène, est adapté pour s'articuler avec une tête, éventuellement prothésée, de l'humérus, non représenté, du patient opéré, en vue de reproduire un comportement articulaire aussi proche que possible du comportement d'origine de l'épaule du patient.

Le corps articulaire 302, d'axe longitudinal X302-X'302, comprend un plateau 321, globalement perpendiculaire à l'axe X302-X'302, délimité par une face supérieure 322 et par une face inférieure 323. La face supérieure 322 constitue une surface articulaire pour l'articulation de l'omoplate avec la tête de l'humérus. La face inférieure 323 du plateau 321 est pourvue d'un pion central 324, centré sur l'axe X302-X'302, dont la surface latérale externe est hexagonale. Le pion central 324 est creux et définit un logement 325 tronconique, dont le diamètre augmente en s'éloignant du plateau 321.

Comme bien visible aux figures 11 et 12, le plateau 321 a une forme allongée, et s'étend le long d'un axe longitudinal Y321-Y'321, perpendiculaire à l'axe X302-X'302. Le pion central 324 est décalé longitudinalement, selon l'axe Y321-Y'321 par rapport au centre C21 du plateau 321.

Le corps articulaire 302 est fabriqué à partir d'un ou deux matériaux synthétiques biocompatibles et adaptés à une articulation partielle ou totale, comme par exemple le polyéthylène, le PEEK ou le polyuréthane.

L'élément central 303 est sensiblement identique à l'élément central 203 de l'implant glénoïdien conforme au troisième mode de réalisation. Ainsi, l'élément central 303 comprend une partie intermédiaire 303.2 tronconique dont le diamètre diminue entre l'extrémité supérieure 333 et l'extrémité inférieure 334 de l'élément central 303. Une surface latérale externe 331 de la partie intermédiaire 303.2 porte un filetage auto-taraudant et est traversée transversalement par des trous 338 qui sont localisés en bas de la partie intermédiaire 303.2. La partie intermédiaire 303.2 est prolongée vers le bas par une portion inférieure 303.3 tronconique, dont la conicité est plus importante que cette de la partie intermédiaire 303.2. La partie intermédiaire 303.2 est prolongée vers le haut par une partie supérieure 303.1 tronconique, dont le grand diamètre est du côté de la partie intermédiaire 303.2. Une rainure périphérique 337 est définie entre la partie intermédiaire 303.2 et la partie supérieure 303.1.

L'élément central 303 est traversé par une ouverture longitudinale 332, qui débouche de part et d'autre de l'élément central 303 et qui communique avec les trous 338. L'ouverture 332 est composée d'une partie supérieure 332.1 de section transversale hexagonale, qui débouche sur l'extrémité supérieure 333 de l'élément central, et d'une partie inférieure 332.2 cylindrique à section circulaire qui relie la partie supérieure 332.1 à l'extrémité inférieure 334 de l'élément central 303.

L'implantation de l'implant glénoïdien 301 est similaire à celle de l'implant glénoïdien 201. La forme hexagonale du pion central 324 du corps articulaire 302 permet de bloquer la rotation du corps articulaire 302 par rapport à la glène.

Une fois l'implant glénoïdien 301 posé, le tissu osseux de la glène peut recoloniser les trous 338 et l'ouverture 332 de l'élément central 303, ce qui contribue à bloquer la rotation de l'élément central 303 par rapport à la glène, et améliore la tenue et la durée de vie de l'implant glénoïdien 301.

Le blocage en rotation de l'élément central 303 par rapport à la glène est effectué partiellement, voire exclusivement, par l'intermédiaire du corps articulaire 302. En effet, grâce à sa section hexagonale du pion central 324, la rotation du corps articulaire 302 par rapport à la glène est bloquée. Compte tenu du fait que le corps articulaire 302 et l'élément central 303 sont tous les deux bloqués en rotation par rapport à la glène, il n'est pas indispensable de prévoir des moyens de blocage de la rotation du corps articulaire 302 par rapport à l'élément central 303. Toutefois, il est envisageable d'effectuer un blocage, par exemple de type tenon et mortaise, de la rotation du corps articulaire 302 par rapport à l'élément central 303.

La section transversale maximale de l'élément central 303, prise perpendiculairement à l'axe longitudinal X303-X'303, est inférieure à la section transversale du plateau 321 du corps articulaire 302, prise perpendiculairement à l'axe longitudinal X302-X'302. Le contact entre l'élément central 303 et le corps articulaire 302 est réalisé uniquement sur une partie du corps articulaire 302, en l'occurrence sur le pion central 324. L'élément central 303 n'est pas en contact avec la totalité de la surface inférieure 323 du plateau 321. L'élément central 303 assure l'ancrage du corps articulaire 302 dans la glène, et la surface inférieure 323 du plateau 321 n'est pas soutenue par l'élément central 303.

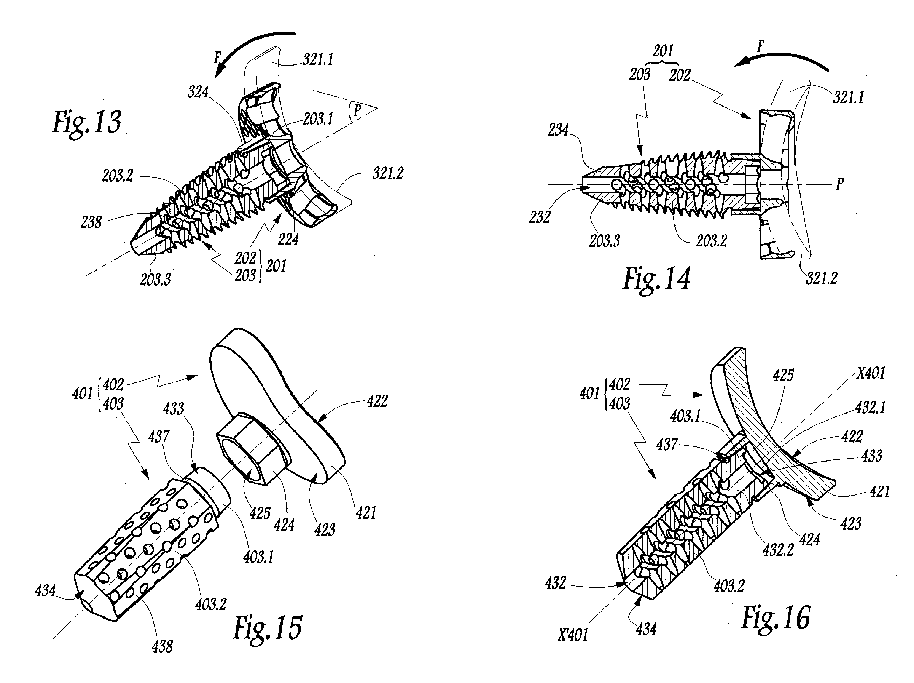

Les figures 13 et 14 représentent le corps articulaire 202 et l'élément central 203 de l'implant glénoïdien 201. Le corps articulaire 302 du quatrième mode de réalisation est superposé avec le corps articulaire 202, et est représenté dans une position dans laquelle il est assemblé avec l'élément central 203.

Le décalage latéral du pion central 324 par rapport à l'axe longitudinal Y-321-Y'321 du plateau 321 est bien visible aux figures 11 et 12, où une partie longue 321.1 du plateau 321, située d'un côté d'un plan P perpendiculaire à l'axe X302-X'302, dépasse latéralement du corps articulaire 202. Une partie courte 321.2 du plateau 321 est située de l'autre côté du plan P. Lorsque le chirurgien implante l'implant glénoïdien 301 dans l'omoplate, il positionne le corps articulaire 301 en orientant le pion central 324 vers le bas, de sorte que lorsque le patient opéré lève le bras, la tête de l'humérus a tendance à faire basculer la partie longue 321.1 du plateau 321 vers l'omoplate, comme représenté par la flèche F. La face inférieure 323 de la partie longue 321.1 est en contact direct avec la glène, puisque le pion 324 est décalé latéralement, ce qui permet avantageusement à la glène d'encaisser directement l'effort de compression qui résulte du basculement de la partie longue 321.1.

Les figures 15 et 16 représentent un implant glénoïdien 401, dont les éléments analogues à ceux du quatrième mode de réalisation portent les mêmes références, augmentées de 100.

L'implant glénoïdien 401 est composé d'un corps articulaire 402, strictement identique au corps articulaire 302 du quatrième mode de réalisation, et d'un élément central 403, de type « press fit », pour l'ancrage du corps articulaire 402 dans la glène de l'omoplate.

Ainsi, le corps articulaire 402, d'axe longitudinal X402-X'402, comprend un plateau 421, dont la face supérieure 422 constitue une surface articulaire pour l'articulation de l'omoplate avec la tête de l'humérus. Une face inférieure 423 du plateau 421 est pourvue d'un pion central 424 de section transversale hexagonale. Le pion central 424 est creux et définit un logement 425 tronconique, dont le diamètre diminue en se rapprochant du plateau 421.

Le corps articulaire 402 est fabriqué à partir d'un ou deux matériaux synthétiques biocompatibles, comme par exemple le polyéthylène, le PEEK ou le polyuréthane.

L'élément central 403, d'axe longitudinal X403-X'403, comprend une partie inférieure 403.2 en forme de prisme à base hexagonale. On note 433 une extrémité supérieure de l'élément central 403, et 434 une extrémité inférieure de l'élément central 403. Les arêtes latérales de l'élément central sont biseautées progressivement en direction de l'extrémité inférieure 434. La partie inférieure 403.2 est traversée transversalement par des trous 438. La partie inférieure 403.2 est prolongée vers le haut par une partie supérieure 403.1 tronconique, dont le diamètre diminue en s'éloignant de la partie supérieure 403.1. Une rainure périphérique 437 est définie entre la partie supérieure 403.1 et la partie inférieure 403.2.

L'élément central 403 est fabriqué à partir d'un matériau métallique ou de céramique biocompatible, tel qu'un acier inoxydable, du titane, un alliage de titane ou un alliage de carbone.

La section transversale maximale de l'élément central 403, prise perpendiculairement à l'axe longitudinal X403-X'403, est inférieure à la section transversale du plateau 421 du corps articulaire 402, prise perpendiculairement à l'axe longitudinal X402-X'402. Le contact entre l'élément central 403 et le corps articulaire 402 est réalisé uniquement sur une partie du corps articulaire 402, en l'occurrence sur le pion central 424. L'élément central 403 n'est pas en contact avec la totalité de la surface inférieure 423 du plateau 421. L'élément central 403 assure l'ancrage du corps articulaire 402 dans la glène, et la surface inférieure 423 du plateau 421 n'est pas soutenue par l'élément central 403.

Lorsqu'il convient de réaliser la pose de l'implant glénoïdien 401 dans la glène de l'omoplate, le chirurgien perce préalablement un trou dans la glène de dimensions légèrement inférieures à celles de l'élément central 403. Puis, le chirurgien impacte l'élément central 403 pour le faire rentrer dans le trou. La pénétration de l'élément central 403 dans la glène est facilitée grâce aux arêtes latérales biseautées. La rotation de l'élément central 403 par rapport à la glène est bloquée grâce à la forme hexagonale de la section transversale de l'élément central 403. La repousse osseuse dans les trous 438 contribue également à bloquer la rotation de l'élément central 403 par rapport à la glène.

Puis, le chirurgien emmanche le pion central 424 du corps articulaire 402 sur la partie supérieure 403.1 de l'élément central 403. Lorsque le corps articulaire 402 est assemblé à l'élément central 403, le corps articulaire 402 est bloqué en translation et en rotation par rapport à l'élément central 403 grâce à la fixation de type « cône-morse » entre ces deux éléments. Les axes X402-X'402 et X403-X'403 sont alors alignés et coïncident avec l'axe longitudinal X401-X'401 de l'implant glénoïdien 401.

Le blocage en rotation du corps articulaire 402 par rapport à la glène est assuré par la section hexagonale du pion central 424, et par les frottements entre la partie supérieure 403.1 de l'élément central 403 et le logement 425 du pion central 424.

Ainsi, l'implant glénoïdien 401 est bloqué en rotation, autour de l'axe X401-X'401, par rapport à l'omoplate.

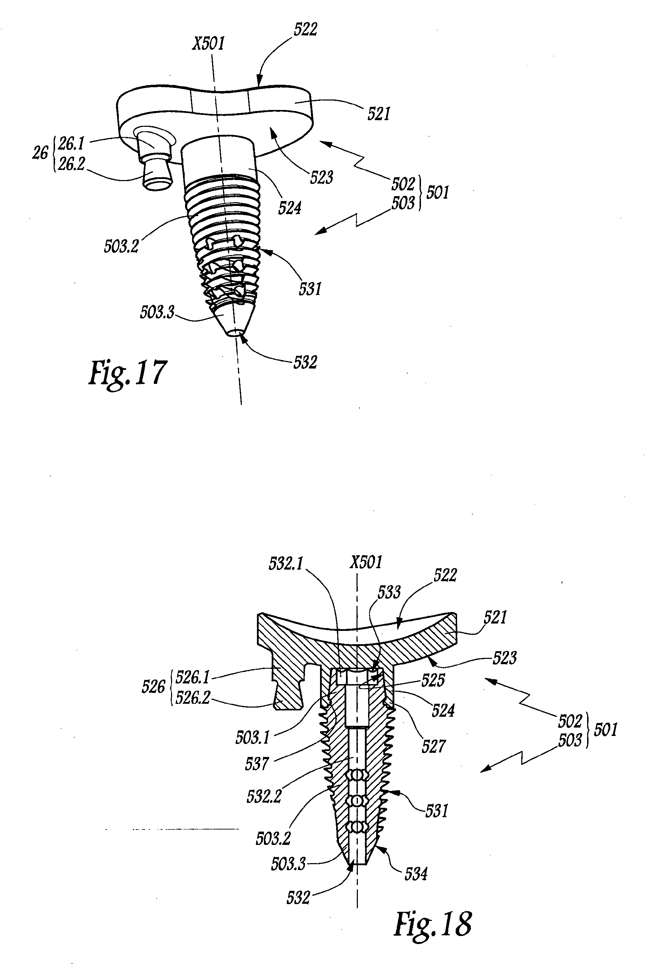

Les figures 17 et 18 représentent un implant glénoïdien 501 dont les éléments analogues à ceux du quatrième mode de réalisation portent les mêmes références, augmentées de 200.

L'implant glénoïdien 501 est composé d'un corps articulaire 502, identique au corps articulaire 302 du quatrième mode de réalisation, et d'un élément central 403 pour l'ancrage du corps articulaire 402 dans la glène de l'omoplate.

Le corps articulaire 502 comprend un plateau 521 délimité par une face supérieure 522 concave, qui constitue une surface articulaire pour l'articulation de l'omoplate avec la tête de l'humérus, et par une face inférieure 523, équipée d'un pion central 524 cylindrique à section circulaire. Le pion central 524 est creux et définit un logement 525 tronconique, dont le diamètre augmente en s'éloignant du plateau 521.

La face inférieure 523 est également équipée d'un plot latéral 526, parallèle au pion central 524, qui comprend une partie supérieure 526.1 cylindrique à section circulaire, de diamètre inférieur au diamètre du pion central 524, qui est reliée à la face inférieure 523 du plateau 521. Le plot latéral 526 comprend également une partie inférieure 526.2 tronconique, reliée à la partie supérieure 526.1, dont le diamètre augmente en s'éloignant du plateau 521. La surface latérale interne du pion central 524 est pourvue d'une ailette périphérique 527.

L'élément central 503 comprend une partie intermédiaire 503.2 tronconique dont le diamètre diminue entre une extrémité supérieure 533 et une extrémité inférieure 534 de l'élément central 503 et qui communique avec les trous 538. Une surface latérale externe 531 de la partie intermédiaire 503.2 porte un filetage auto-taraudant et est traversée transversalement par des trous 538 qui sont localisés en bas de la partie intermédiaire 503.2. La partie intermédiaire 503.2 est prolongée vers le bas par une portion inférieure 503.3 tronconique, dont la conicité est plus importante que celle de la partie intermédiaire 503.2. La partie intermédiaire 503.2 est prolongée vers le haut par une partie supérieure 503.1 tronconique, dont le grand diamètre est du côté de la partie intermédiaire 503.2. Une rainure périphérique 537 est creusée au niveau de la jonction entre la partie supérieure 503.1 et la partie intermédiaire 503.2.

L'élément central 503 est traversé par une ouverture longitudinale 532, qui débouche de part et d'autre de l'élément central 503 et qui communique avec les trous 538. L'ouverture 532 est composée d'une partie supérieure 532.1 de section transversale hexagonale, qui débouche sur l'extrémité supérieure 533 de l'élément central, et d'une partie inférieure 532.2 cylindrique à section circulaire qui relie la partie supérieure 532.1 à l'extrémité inférieure 534 de l'élément central 503.

La section transversale maximale de l'élément central 503 est inférieure à la section transversale du plateau du corps articulaire 202. Le contact entre l'élément central 503 et le corps articulaire 502 est réalisé uniquement sur une partie du corps articulaire 502, en l'occurrence sur le pion central 524. L'élément central 503 n'est pas en contact avec la totalité de la surface inférieure 523 du plateau 521. L'élément central 503 assure l'ancrage du corps articulaire 502 dans la glène, et la surface inférieure 523 du plateau 521 n'est pas soutenue par l'élément central 503.

Lorsqu'il convient d'implanter l'implant glénoïdien 501 dans la glène d'un patient, le chirurgien perce préalablement un trou de dimensions légèrement inférieures aux dimensions de l'élément central 503. Le chirurgien perce également un trou de réception du plot latéral 526. Puis, le chirurgien visse l'élément central 503 dans le trou, au moyen d'un outil équipé d'un embout à section hexagonale qu'il insère dans la partie supérieure 532.1 de l'ouverture 532 de l'élément central 503.

Puis, le chirurgien clipse le corps articulaire 502 sur la partie supérieure 503.1 de l'élément central. L'ailette périphérique 527 se positionne alors dans la rainure 537 de l'élément central 503, ce qui permet de bloquer la translation du corps articulaire 502 par rapport à l'élément central. Le plot latéral 526 permet de bloquer la rotation du corps articulaire 502 par rapport à la glène.

Une fois l'implant glénoïdien 501 posé, le tissu osseux de la glène peut recoloniser les trous 538 et l'ouverture 532 de l'élément central 503, ce qui contribue à bloquer la rotation de l'élément central 503 par rapport à la glène et améliore la tenue de l'implant glénoïdien 501.

On remarque que les éléments centraux des implants glénoïdiens conformes aux troisième à sixième modes de réalisation de l'invention sont conçus pour pouvoir recevoir indifféremment un corps articulaire métallique ou céramique et un corps articulaire synthétique. En effet, l'élément central 203 de l'implant glénoïdien 201 permet la fixation d'un corps articulaire métallique ou céramique, au moyen de la partie supérieure 203.1 qui constitue un cône d'emmanchement. De plus, la rainure 237 de l'élément central 203 permet de clipser un corps articulaire synthétique pourvu d'une ailette périphérique semblable à l'ailette 527 du corps articulaire 502. De même, les éléments centraux 303, 403 et 503 sont pourvus, à même effet, d'une partie tronconique 303.1, 403.1, 503.1, destinée à être emmanchée dans un logement tronconique 325, 425, 525 défini par un corps articulaire métallique ou céramique, et d'une rainure 337, 437, 537 de clipsage d'un corps articulaire synthétique.

Dans une variante du premier mode de réalisation, le taraudage de la partie taraudée 32.2 est remplacé par des rainures réparties le long de l'axe X3-X'3. D'autre part, le nombre d'ailettes 25 peut varier.

Dans une variante du premier mode de réalisation, le corps articulaire 2 est réalisé dans un matériau dur métallique ou en céramique, tel que le pyrocarbone. Ce matériau n'étant pas prévu pour se déformer, la fixation entre le corps articulaire 2 et l'élément central 3 est, par exemple, de type cône morse. Cette fixation remplace les ailettes 25. On notera que le PEEK présente une rigidité relativement importante pour un polymère. Ainsi, il est envisageable d'utiliser un emmanchement de type cône morse pour fixer un élément en PEEK.

De même, il est envisageable de fabriquer les corps articulaires 302 et 402 dans un matériau dur métallique ou en céramique, tel que le pyrocarbone.

Dans une variante supplémentaire du premier mode de réalisation, la partie supérieure 32.1 de l'ouverture 32 de l'élément central 3 ainsi que la partie supérieure 24.1 1 sont tronconiques au lieu d'être cylindriques.

Dans une autre variante non représentée du premier mode de réalisation, le filetage de la surface latérale externe 31 de l'élément central 3 est à gauche, tandis que le filetage de la tige 44 de la vis 4 est à droite. Dans ce cas, le filetage de la tête 41 de la vis 4 est à droite et le taraudage de la partie inférieure 32.4 de l'ouverture 32 est également à droite.

Dans une autre variante non représentée du deuxième mode de réalisation, le filetage de la surface latérale externe 131 de l'élément central 103 est à gauche, tandis que le filetage de la tige 144 de la vis 104 est à droite.

En variante, dans le deuxième mode de réalisation, la partie 132.1 de l'ouverture 132 de l'élément central 103 est tronconique et la partie 132.2 est cylindrique.

Dans une variante des premier et second modes de réalisation, les vis 4 et 104 sont en polymère, par exemple en PEEK.

Dans une variante des premier, second et cinquième modes de réalisation, les éléments centraux 3, 103 et 403 sont en polymère, par exemple en PEEK.

Dans une variante du deuxième mode de réalisation, la partie inférieure 124.2 du pion central 124 est cylindrique à section circulaire. Dans ce cas, la partie 132.2 est également cylindrique à section circulaire et est adaptée pour recevoir la partie inférieure 124.2 du pion central 124, de sorte que la fixation du corps articulaire 102 est réalisée en enfonçant en force le pion central 124 dans l'élément central 103.

Dans une variante, la section transversale du pion central est en forme d'un polygone qui a un nombre de côtés différent de quatre. Par exemple, le pion central peut être triangulaire ou octogonal.

En variante non représentée, un élément central définit un logement de réception d'un pion central d'un corps articulaire. Par exemple, pour être apte à recevoir un corps articulaire synthétique, le logement d'un élément central peut comporter un taraudage interne, similaire au taraudage de la partie taraudée 32.2 de l'élément central 32, dans laquelle se clipsent des ailettes prévues le long d'un pion central d'un corps articulaire. En complément, pour être apte à recevoir un corps articulaire métallique ou céramique, l'élément central peut comporter un logement tronconique de réception d'un pion central tronconique d'un corps articulaire.

En variante, dans le premier mode de réalisation, la partie hexagonale 32.3 de l'élément central 3 et le trou borgne 42 de la tête de la vis 4 peuvent être à section carrée, en forme de croix, une empreinte Torx (marque déposée), ou de toute autre géométrie permettant une prise d'outil. Il en va de même pour le second mode de réalisation en ce qui concerne le trou borgne 142 de la vis 104 et pour la partie hexagonale 132.3 de l'élément central 103. L'embout des outils utilisés sont bien évidemment adaptés aux empreintes des éléments à visser.

Dans une variante, les corps articulaires 102 et 202 sont équipés de deux douilles 105 supplémentaires logées dans les logements 121 a et 121 c ou 221 a et 221 c.

En outre, les différents modes de réalisation et les variantes décrites ci-dessus peuvent être, partiellement ou totalement, combinés entre eux pour donner lieu à d'autres modes de réalisation.

## Revendications

1. Implant glénoïdien (1 ; 101 ; 201 ; 301 ; 401 ; 501) pour prothèse d'épaule, destiné à être implanté dans la glène d'une omoplate, comprenant un corps articulaire (2 ; 102 ; 202 ; 302 ; 402 ; 502), pour l'articulation avec l'humérus, et un élément central (3 ; 103 ; 203 ; 303 ; 403 ; 503) de fixation du corps articulaire (2 ; 102 ; 202 ; 302 ; 402 ; 502) dans la glène,
le corps articulaire (2 ; 102 ; 202 ; 302 ; 402 ; 502) comprenant un plateau (21 ; 121 ; 221 ; 321 ; 421 ; 521) dont une face (23 ; 123 ; 223 ; 323 ; 423 ; 523), destinée à être tournée vers la glène, est pourvue d'un pion central (24 ; 124 ; 224; 324 ; 424 ; 524) d'assemblage du corps articulaire (2 ; 102 ; 202 ; 302 ; 402 ; 502) avec l'élément central (3 ; 103 ; 203 ; 303 ; 403 ; 503),
l'élément central (3 ; 103 ; 203 ; 303 ; 403 ; 503) étant pourvu de moyens d'assemblage (32.1, 32.2 ; 132.1, 132.2 ; 203.1, 237 ; 303.1, 337 ; 403.1, 437 ; 503.1, 537) avec le pion central (24 ; 124 ; 224 ; 324 ; 424 ; 524) et
l'implant glénoïdien (1 ; 101 ; 201 ; 301 ; 401 ; 501) comprenant des premiers moyens (31, 4 ; 131, 104 ; 238 ; 338 ; 403.2, 438 ; 538) de blocage de la rotation de l'élément central (3 ; 103 ; 203 ; 303 ; 403 ; 503) par rapport à la glène,
l'implant glénoïdien comprenant des deuxièmes moyens (26 ; 106.; 324 ; 424 ; 526) de blocage de la rotation du corps articulaire (2 ; 102 ; 202 ; 302 ; 402 ; 502) par rapport à la glène, les premiers moyens de blocage de la rotation comprenant une vis (4 ; 104), **caractérisé en ce que** l'élément central (3 ; 103) est traversé longitudinalement par une ouverture (32 ; 132) de passage de la vis (4 ; 104), et **en ce que** la tige (44 ; 144) de la vis (4 ; 104) est filetée dans un sens opposé à un filetage porté par la surface latérale externe (31 ; 131) de l'élément central (3 ; 103):

2. Implant glénoïdien (1) selon la revendication 1, **caractérisé en ce que** la tête (41) de la vis (4) est filetée dans le même sens que la tige (44) de la vis (4).

3. Implant glénoïdien (1) selon la revendication 2, **caractérisé en ce que** le pas du filetage de la tête (41) de la vis (4) est égal au pas du filetage de la tige (44) de la vis (4).

4. Implant glénoïdien (301 ; 401) selon l'une des revendications précédentes, **caractérisé en ce que** les deuxièmes moyens de blocage de la rotation comprennent une surface latérale externe du pion central (324 ; 424) de section transversale polygonale.

5. Implant glénoïdien (201 ; 301 ; 401 ; 501) selon l'une des revendications précédentes, **caractérisé en ce que** les moyens d'assemblage (203.1, 237 ; 303.1, 337 ; 403.1, 437 ; 503.1, 537) sont adaptés pour coopérer indifféremment avec un corps articulaire (202) métallique ou céramique et avec un corps articulaire (302 ; 402 ; 502) synthétique.

6. Implant glénoïdien (201 ; 301 ; 401 ; 401 ; 501) selon la revendication 5, **caractérisé en ce que** les moyens d'assemblage comprennent une partie tronconique (203.1 ; 303.1 ; 403.1 ; 503.1) ou un logement tronconique (132.2), adapté pour coopérer par emmanchement, notamment de type cône morse, avec, respectivement, un logement tronconique (225 ; 325 ; 425 ; 525) ou une partie tronconique (124.2) du pion central (224 ; 324 ; 424 ; 524).

7. Implant glénoïdien (201 ; 301 ; 401 ; 501) selon l'une des revendications 5 et 6, **caractérisé en ce que** les moyens d'assemblage comprennent une ailette périphérique (527) ou une rainure périphérique (32.2), adaptée pour coopérer par déformation souple et complémentarité de formes avec, respectivement, une rainure (537) ou une ailette (25) du pion central (224 ; 324 ; 424 ; 524).

8. Implant glénoïdien (1 ; 301 ; 401 ; 501) selon l'une des revendications précédentes, **caractérisé en ce que** le plateau (21 ; 321 ; 421 ; 521) est de forme allongée et **en ce que en ce que** le pion central (24 ; 324 ; 424 ; 524) est décalé, selon un axe longitudinal (Y321-Y'321) du plateau (21 ; 321 ; 421 ; 521), par rapport au centre (C21) du plateau (21 ; 321 ; 421 ; 521).

9. Implant glénoïdien (1 ; 101 ; 201 ; 301 ; 401 ; 501) selon l'une des revendications précédentes, **caractérisé en ce que** la section transversale du plateau (21 ; 121 ; 221 ; 321 ; 421 ; 521) est supérieure à la section transversale de l'élément central (3 ; 103 ; 203 ; 303 ; 403 ; 503).

10. Implant glénoïdien (1 ; 101 ; 201 ; 301 ; 401 ; 501) selon la revendication 9, **caractérisé en ce que** la section transversale du plateau (21 ; 121 ; 221 ; 321 ; 421 ; 521) est entre 1,8 à 5 fois supérieure à la section transversale de l'élément central (3 ; 103 ; 203 ; 303 ; 403 ; 503), de préférence 2,2 fois supérieure à la section transversale de l'élément central (3 ; 103 ; 203 ; 303 ; 403 ; 503).

11. Kit chirurgical, **caractérisé en ce qu'**il comprend au moins un élément central (202 ; 203 ; 303 ; 403 ; 503) appartenant à un implant glénoïdien (201 ; 301 ; 401 ; 501) selon la revendication 5, au moins un corps articulaire (202) métallique ou céramique appartenant à un implant glénoïdien (201 ; 301 ; 401 ; 501) selon la revendication 6 et au moins un corps articulaire (302 ; 402 ; 502) synthétique, appartenant à un implant glénoïdien (201 ; 301 ; 401 ; 501) selon la revendication 7.

## Patentansprüche

1. Glenoidimplantat (1; 101; 201; 301; 401; 501) für Schulterprothese, welches dazu vorgesehen ist, in die Gelenkpfanne eines Schulterblatts implantiert zu werden, mit einem Gelenkkörper (2; 102; 202; 302; 402; 502) für das Gelenk mit dem Oberarmknochen und einem zentralen Element (3; 103; 203; 303; 403; 503) zum Fixieren des Gelenkkörpers (2; 102; 202; 302; 402; 502) in der Gelenkpfanne,
wobei der Gelenkkörper (2; 102; 202; 302; 402; 502) eine Platte (21; 121; 221; 321; 421; 521) aufweist, von welcher eine Fläche (23; 123; 223; 323; 423; 523), welche dazu vorgesehen ist, hin zu der Gelenkpfanne zugewandt zu sein, mit einem zentralen Zapfen (24; 124; 224; 324; 424; 524) zum Zusammenbauen des Gelenkkörpers (2; 102; 202; 302; 402; 502) mit dem zentralen Element (3; 103; 203; 303; 403; 503) versehen ist,
wobei das zentrale Element (3; 103; 203; 303; 403; 503) mit Mitteln zum Zusammenbauen (32.1, 32.2; 132.1, 132.2; 203.1, 237; 303.1, 337; 403.1, 437; 503.1, 537) mit dem zentralen Zapfen (24; 124; 224; 324; 424; 524) versehen ist, und
wobei das Glenoidimplantat (1; 101; 201; 301; 401; 501) erste Mittel (31, 4; 131, 104; 238; 338; 403.2, 438; 548) zum Blockieren der Drehung des zentralen Elements (3; 103; 203; 303; 403; 503) bezüglich der Gelenkpfanne aufweist,
wobei das Glenoidimplantat zweite Mittel (26; 106; 324; 424; 526) zum Blockieren der Drehung des Gelenkkörpers (2; 102; 202; 302; 402; 502) bezüglich der Gelenkpfanne aufweist, wobei die ersten Mittel zum Blockieren der Drehung eine Schraube (4; 104) aufweisen, **dadurch gekennzeichnet, dass** durch das zentrale Element (3; 103) der Länge nach eine Öffnung (32; 132) zum Durchlassen der Schraube (4:104) hindurchverläuft und dass der Schaft (44; 144) der Schraube (4;104) gegensinnig zu einem Gewinde, welches sich an einer seitlichen, äußeren Fläche (31; 131) des zentralen Elements (3; 103) befindet, mit einem Gewinde versehen ist.

2. Glenoidimplantat (1) gemäß Anspruch 1, **dadurch gekennzeichnet, dass** der Kopf (41) der Schraube (4) in die gleiche Richtung wie der Schaft (44) der Schraube (4) mit einem Gewinde versehen ist.

3. Glenoidimplantat (1) gemäß Anspruch 2, **dadurch gekennzeichnet, dass** die Steigung des Gewindes des Kopfs (41) der Schraube (4) gleich der Steigung des Gewindes des Schafts (44) der Schraube (4) ist.

4. Glenoidimplantat (301; 401) gemäß einem der vorgehenden Ansprüche, **dadurch gekennzeichnet, dass** die zweiten Mittel zum Blockieren der Drehung eine seitliche, äußere Fläche des zentralen Zapfens (324; 424) mit einem vieleckigen Querschnitt aufweisen.

5. Glenoidimplantat (201; 301; 401; 501) gemäß einem der vorgehenden Ansprüche, **dadurch gekennzeichnet, dass** die Mittel zum Zusammenbauen (203.1, 237; 303.1, 337; 403.1, 437; 503.1, 537) dazu eingerichtet sind, ohne Unterschied mit einem metallischen oder keramischen Gelenkkörper (202) und mit einem synthetischen Gelenkkörper (302; 402; 502) zusammenzuwirken.

6. Glenoidimplantat (201; 301; 401; 401; 501) gemäß dem Anspruch 5, **dadurch gekennzeichnet, dass** die Mittel zum Zusammenbauen einen kegelstumpfförmigen Abschnitt (203.1; 303.1; 403.1; 503.1) oder einen kegelstumpfförmigen Sitz (132.2) aufweisen, welcher dazu eingerichtet ist, durch Einpassen, insbesondere vom Morsekegel-Typ, jeweilig mit einem kegelstumpfförmigen Sitz (225; 325; 425; 525) oder einem kegelstumpfförmigen Abschnitt (124.2) des zentralen Zapfens (224; 324; 424; 525) zusammenzuwirken.

7. Glenoidimplantat (201; 301; 401; 501) gemäß einem der Ansprüche 5 und 6, **dadurch gekennzeichnet, dass** die Mittel zum Zusammenbauen eine Umfangsrippe (527) oder eine Umfangsnut (32.2) aufweisen, welche dazu eingerichtet ist, jeweils durch flexible Verformung und Komplementarität der Formen mit einer Nut (537) oder einer Rippe (25) des zentralen Zapfens (224; 324; 424; 524) zusammenzuwirken.

8. Glenoidimplantat (201; 301; 401; 501) gemäß einem der vorhergehenden Ansprüche, **dadurch gekennzeichnet, dass** die Platte (21; 321; 421; 521) von einer langgestreckten Form ist und dass der zentrale Zapfen (24; 324; 424; 524) entlang einer Längsachse (Y321-Y321') der Platte (21; 321; 421; 521) bezüglich der Mitte (C21) der Platte (21; 321; 421; 521) versetzt ist.

9. Glenoidimplantat (1; 101; 201; 301; 401; 501) gemäß einem der vorhergehenden Ansprüche, **dadurch gekennzeichnet, dass** der Querschnitt der Platte (21; 121; 221; 321; 421; 521) größer als der Querschnitt des zentralen Elements (3; 103; 203; 303; 403; 503) ist.

10. Glenoidimplantat (1; 101; 201; 301; 401; 501) gemäß Anspruch 9, **dadurch gekennzeichnet, dass** der Querschnitt der Platte (21; 121; 221; 321; 421; 521) zwischen 1,8 bis 5 Mal größer als der Querschnitt des zentralen Elements (3; 103; 203; 303; 403; 503), bevorzugt 2,2 Mal größer als der Querschnitt des zentralen Elements (3; 103; 203; 303; 403; 503) ist.

11. Chirurgie-Set, **dadurch gekennzeichnet, dass** es wenigstens ein zentrales Element (203; 303; 403; 503), welches zu einem Glenoidimplantat (201; 301; 401; 501) gemäß Anspruch 5 gehört, wenigstens einen Gelenkkörper (202) aus Metall oder Keramik, welcher zu einem Glenoidimplantat (201; 301; 401; 501) gemäß Anspruch 6 gehört, und wenigstens einen synthetischen Gelenkkörper (302; 402; 502) aufweist, welcher zu einem Glenoidimplantat (201; 301; 401; 501) gemäß Anspruch 7 gehört.

## Claims

1. Glenoid implant (1; 101; 201; 301; 401; 501) for a shoulder prosthesis intended to be implanted into the glenoid cavity of a scapula comprising an articular body (2; 102; 202; 302; 402; 502) for articulation with the humerus and a central element (3; 103; 203; 303; 403; 503) for fixture of the articular body (2; 102; 202; 302; 402; 502) in the glenoid cavity,
wherein the articular body (2; 102; 202; 302; 402; 502) comprises a plate (21; 121; 221; 321; 421; 521), of which one face (23; 123; 223; 323; 423; 523) intended to be turned towards the glenoid cavity is provided with a central pin (24; 124; 224; 324; 424; 524) for connecting the articular body (2; 102; 202; 302; 402; 502) to the central element (3; 103; 203; 303; 403; 503),
and the central element (3; 103; 203; 303; 403; 503) is provided with elements (32.1, 32.2; 132.1, 132.2; 203.1, 237; 303.1, 337; 403.1, 437; 503.1, 537) for connecting to the central pin (24; 124; 224; 324; 424; 524) and
wherein the glenoid implant (1; 101; 201; 301; 401; 501) comprises first elements (31, 4; 131, 104; 238; 338; 403.2, 438; 538) for blocking the rotation of the central element (3; 103; 203; 303; 403; 503) in relation to the glenoid cavity,
and the glenoid implant comprises second elements (26; 106; 324; 424; 526) for locking the rotation of the articular body (2; 102; 202; 302; 402; 502) in relation to the glenoid cavity, wherein the first rotation blocking elements comprise a screw (4; 104), **characterised in that** the central element (3; 103) has an opening (32; 132) passing longitudinally through it for passage of the screw (4; 104), and that the stem (44; 144) of the screw (4; 104) is threaded in an opposed direction to a thread borne by the outer side surface (31; 131) of the central element (3; 103).

2. Glenoid implant (1) according to claim 1, **characterised in that** the head (41) of the screw (4) is threaded in the same direction as the stem (44) of the screw (4).

3. Glenoid implant (1) according to claim 1, **characterised in that** the pitch of the thread of the head (41) of the screw (4) is equal to the pitch of the thread of the stem (44) of the screw (4).

4. Glenoid implant (301; 401) according to one of the preceding claims, **characterised in that** the second rotation blocking elements include an outer side surface of the central pin (324; 424) with a polygonal cross-section.

5. Glenoid implant (201; 301; 401; 501) according to one of the preceding claims, **characterised in that** the connecting elements (203.1, 237; 303.1, 337; 403.1, 437; 503.1, 537) are adjusted to cooperate equally with a metal or ceramic articular body (202) and with an articular body (302; 402; 502) of synthetic material.

6. Glenoid implant (201; 301; 401; 501) according to claim 5, **characterised in that** the connecting elements comprise a tapered part (203.1; 303.1; 403.1; 503.1) or a tapered cavity (132.2) suitable for cooperating by friction fit, in particular by machine taper, respectively with a tapered cavity (225; 325; 425; 525) or a tapered part (124.2) of the central pin (224; 324; 424; 524).

7. Glenoid implant (201; 301; 401; 501) according to one of claims 5 and 6, **characterised in that** the connecting elements comprise a peripheral rib (527) or a peripheral groove (32.2) adjusted to cooperate by flexible deformation and shape complementarity respectively with a groove (537) or a rib (25) of the central pin (224; 324; 424; 524).

8. Glenoid implant (1; 301; 401; 501) according to one of the preceding claims, **characterised in that** the plate (21; 321; 421; 521) has an elongated shape and that the central pin (24; 324; 424; 524) is shifted along a longitudinal axis (Y321-Y'321) of the plate (21; 321; 421; 521) in relation to the centre (C21) of the plate (21; 321; 421; 521).

9. Glenoid implant (1; 101; 201; 301; 401; 501) according to one of the preceding claims, **characterised in that** the cross-section of the plate (21; 121; 221; 321; 421; 521) is larger than the cross-section of the central element (3; 103; 203; 303; 403; 503).

10. Glenoid implant (1; 101; 201; 301; 401; 501) according to claim 9, **characterised in that** the cross-section of the plate (21; 121; 221; 321; 421; 521) is between 1.8- and 5-times the size of the cross-section of the central element (3; 103; 203; 303; 403; 503), preferably 2.2-times the size of the cross-section of the central element (3; 103; 203; 303; 403; 503).

11. Surgical kit, **characterised in that** it comprises at least one central element (202; 203; 303; 403; 503) belonging to a glenoid implant (201; 301; 401; 501) according to claim 5, at least one metal or ceramic articular body (202) belonging to a glenoid implant (201; 301; 401; 501) according to claim 6 and at least one articular body (302; 402; 502) of synthetic material belonging to a glenoid implant (201; 301; 401; 501) according to claim 7.
